# EUROPEAN PATENT SPECIFICATION

(11) **EP 1 755 676 B1**
(45) Date of publication and mention of the grant of the patent: **28.11.2012**
(21) Application number: 05714425.5
(22) Date of filing: 08.02.2005
(51) Int. Cl.: A61K 41/00, A61N 5/06, A61P 17/00, A61P 17/10, A61K 31/409, A61K 31/555, A61K 31/203, A61K 31/07

(54) **PHOTODYNAMIC THERAPY FOR THE TREATMENT OF HYPERACTIVE SEBACEOUS GLAND DISORDERS USING TOPICALLY APPLIED VERTEPORFIN AND/OR LEMUTEPORFIN**
PHOTODYNAMISCHE THERAPIE ZUR BEHANDLUNG VON TALGDRÜSENHYPERAKTIVITÄT MIT TOPISCH AUFGEBRACHTEM VERTEPORFIN UND/ODER LEMUTEPORFIN
THÉRAPIE PHOTODYNAMIQUE POUR LE TRAITEMENT D'AFFECTIONS DES GLANDES SÉBACÉES HYPERACTIVES EN UTILISANT VERTEPORFIN ET/OU LEMUTEPORFIN

(30) Priority: 09.06.2004 CA 2470403
(43) Date of publication of application: 28.02.2007
(73) Proprietor: QLT, Inc., Vancouver, British Columbia V5T 4T5 (CA)
(72) Inventor: CURAUDEAU, Alain, H., West Vancouver, British Columbia V7V 1S8 (CA); NEYNDORFF, Herma, C., Vancouver, British Columbia V5L 2Y8 (CA); TAO, Jing-Song, Vancouver, British Columbia V6L 1B1 (CA); LEVY, Julia, G., Vancouver, British Columbia V6J 4Z3 (CA); HUNT, David, W., C., Surrey, British Columbia V4A 2X1 (CA)
(74) Representative: Lau, Sarah Jane
(86) International application number: PCT/CA2005/000154
(87) International publication number: WO 2005/120572

(56) References cited:
- WO-A1-03/039597
- WO-A1-03/039597
- WO-A1-2005/074987
- WO-A2-03/017824
- WO-A2-03/017824
- US-A1- 2002 156 062
- US-A1- 2003 050 678
- US-A1- 2003 050 678
- US-B1- 6 600 951
- US-B2- 6 492 420
- GOLLNICK H ET AL: "Management of acne: A report from a global alliance to improve outcomes in acne", JOURNAL OF THE AMERICAN ACADEMY OF DERMATOLOGY 20030701 US LNKD- DOI:10.1067/MJD.2003.618, vol. 49, no. 1 SUPPL., 1 July 2003 (2003-07-01), pages S1-S37, XP002609626, ISSN: 0190-9622
- PIERRE M.B. ET AL: 'Stratum corneum lipids liposome for the topical delivery of 5-aminoLevulinic acid in photodynamic therapy of skin cancer' BMC DERMATOL 2001, pages 1 - 5, XP021016966
- CASAS A. ET AL: 'The Influence of the Vehicle on the Synthesis of Porphyrins after Topical Application of 5-aminolaevulinic acid. Implications in cutaneous photodynamic sensitization' BR. J. DERMATOL vol. 143, no. 3, 2000, pages 564 - 572, XP008112827
- CASAS A. ET AL: 'ALA and ALA hexyl ester in free and liposomal formulations for the photosensitisation of tumour organ cultures' BR. J. CANCER vol. 86, no. 5, 2002, pages 837 - 842, XP008112042
- DIVARIS D.X. ET AL: 'Phototoxic damage to sebaceous glands and hair follicles of mice after systemic administration of 5-aminolevulinic acid correlates with localized protoporphyrinIX fluorescence' AM J PATHOL. vol. 136, no. 4, 1990, pages 891 - 897, XP008112832
- MORDON S. ET AL: 'Site-specific methylene blue delivery to pilosebaceous structures using highly porous nylon microspheres: an experimental evaluation' LASERS SURG MED. vol. 33, no. 2, 2003, pages 119 - 125
- CHARAKIDA A. ET AL: 'Phototherapy in the Treatment of Acne Vulgaris: What is its Role?' AM. J. CLIN. DERMATOL. vol. 5, no. 4, 2004, pages 211 - 216, XP008112829
- TAUB A.F.: 'Photodynamic therapy in dermatology: history and horizons' J. DRUGS DERMATOL vol. 3, no. 1, 2004, pages S8 - 25, XP008112831
- CEILLEY R.I.: 'Advances in the topical treatment of acne and rosacea' J. DRUGS DERMATOL. vol. 3, no. 5, 2004, pages S12 - 22, XP008112834

## Description

### FIELD OF THE INVENTION

This invention relates to a photosensitizer composition as defined in the claims for use in photodynamic therapy (PDT) for treating hyperactive sebaceous gland disorders selected from seborrhea, seborrheic dermatitis, and sebaceous gland hyperplasia.

### BACKGROUND OF THE INVENTION

Hyperactive sebaceous gland disorders such as acne are a common dermatological condition affecting many people. Although often transitory in nature, acne can be associated with long-term consequences such as psychological and/or physical scarring. Clinical manifestations of acne include comedones for mild lesions, papules, pustules, and nodules for more severe inflammatory lesions. The pathogenesis of acne is multi-factorial. It can involve an increase in keratinocytes, desquamation, hyperactive sebaceous glands with increased sebum production, *Propionibacteriumacnes* proliferation and local inflammatory responses.

There are an array of therapies for acne targeting different and in some cases multiple pathogenic factors. Topical agents such as retinoids and benzoyl peroxide can be used for treating mild to moderate acne and are known to be able to remove comedones, kill bacteria and reduce inflammation. Antibiotics, given either topically or orally, can be used for treating mild to moderate acne. Light-based treatments such as 420-nm blue light or 1450-nm thermal lasers can also be used to treat mild to moderate acne. Accutane™ is an orally administrated retinoic acid that has been approved for treating severe, recalcitrant and nodular acne. It can be efficacious at removing comedones, reducing inflammation and inhibiting proliferation, differentiation and lipogenesis of sebaceous glands.

However, there are significant deficiencies associated with currently available therapies. Topical therapies are only marginally effective against mild to moderate acne and can be associated with local irritation. The use of antibiotics is associated with development of drug-resistant bacteria.

Accutane is a known teratogenic agent and is associated with multiple significant systemic toxicities including increased risk of depression, increase in blood lipid and significant mucocutaneous adverse effects. Therefore, there is a need for novel therapeutic approaches with good efficacy and safety profiles.

Photodynamic therapy (PDT) has been proposed as a possible treatment for acne. For example, US Patent Number 5,095,030 (Levy) mentions acne as a possible indication which may be treated with PDT. Other disclosures which mention acne as a possible indication for treatment with PDT include WO03/86460 (Geronemus), WO03/39597 (Boch), WO02/13788 (Anderson), US2001/0023363 (Harm), US6645230 (Whitehurst), US6626932 (Whitehurst), and US5955490 (Kennedy). A more detailed discussion can be found in "Topical ALA-Photodynamic Therapy for the Treatment of Acne Vulgaris" J. Invest Dermatol 115:183-192, 2000. This paper discusses the use of the photosensitizer ALA to treat acne vulgaris. However, the paper discusses serious adverse events that occurred during and after the treatment including erythema, edema, and sensations of pain, burning and itching.

US2003/0050678 (Sierra) and WO03/017824 (MCDaniel) disclose phototherapy of skin disorders by targeting the sebaceous glands.

Citation of the above documents is not intended as an admission that any of the foregoing is pertinent prior art. All statements as to the date or representation as to the contents of these documents is based on the information available to the applicant and does not constitute any admission as to the correctness of the dates or contents of these documents.

### SUMMARY OF THE INVENTION

The present invention provides the use of a hydrophobic and/or lipophilic photosensitizer composition in the manufacture of a medicament for the treatment of a hyperactive sebaceous gland disorder selected from seborrhea, seborrheic dermatitis, and sebaceous gland hyperplasia, wherein said medicament is administered by a method comprising:
(i) topically applying said medicament to skin tissue exhibiting symptoms of a hyperactive sebaceous gland disorder selected from seborrhea, seborrheic dermatitis, and sebaceous gland hyperplasia, and
(ii) exposing the tissue to energy at a wavelength capable of activating the photosensitizer, wherein the photosensitizer is selected from verteporfin, lemuteporfin, and combinations thereof.

The present invention also provides a hydrophobic and/or lipophilic photosensitizer composition for use in the treatment of a hyperactive sebaceous gland disorder selected from seborrhea, seborrheic dermatitis, and sebaceous gland hyperplasia, wherein the photosensitizer is selected from verteporfin, lemuteporfin , and combinations thereof.

The present invention also provides the use of a hydrophobic and/or lipophilic photosensitizer composition in the preparation of a medicament for the treatment of a hyperactive sebaceous gland disorder selected from seborrhea, seborrheic dermatitis, and sebaceous gland hyperplasia, wherein the photosensitizer is selected from verteporfin, lemuteporfin and combinations thereof.

While not wishing to be bound by theory, it is believed that PDT works to treat hyperactive sebaceous gland disorders through at least two mechanisms. First, PDT has an antibacterial effect and, second, it reduces the size and/or activity of the sebaceous glands.

As used herein the term "hydrophobic photosensitizer" refers to photosensitizers that repel water, have a tendency not to combine with water, or are incapable of being substantially dissolved in water. As used herein the term "lipophilic photosensitizer" refers to photosensitizers that have an affinity for, tend to combine with, or are capable of substantially dissolving in, lipids.

One measure of hydrophobicity is the LogP value. In general, substances having a LogP of 0 or greater are thought to be hydrophobic while those with a negative LogP value are thought to be hydrophilic. It is preferred that the photosensitizer compositions used herein have a LogP of not less than 0, preferably not less than 0.5, more preferably not less than 0.75, even more preferably not less than 1.0.

As used herein the term "hydrophobic or lipophilic photosensitizer composition" refers to the composition as it is applied to the affected skin. Therefore, the term encompasses both hydrophobic or lipophilic photosensitizers and hydrophilic photosensitizers that are formulated such that the composition as it is applied to the affected skin is hydrophobic or lipophilic as defined above.

As used herein the term "topically" means applying directly to the skin.

### DETAILED DESCRIPTION OF THE INVENTION

The present invention involves the use of a hydrophobic and/or lipophilic photosensitizer composition in the manufacure of a medicament for the photodynamic treatment of a hyperactive sebaceous gland disorder selected from seborrhea, seborrheic dermatitis, and sebaceous gland hyperplasia wherein the photosensitizer is selected from verteporfin, lemuteporfin, and combinations thereof. The treatment involves the administration of photosensitizer to affected skin and subsequent exposure of that skin to energy of a wavelength capable of activating the photosensitizer. The composition of the invention can also be used in a prophylactic treatment for skin that is suspected of being vulnerable to hyperactive sebaceous gland disorders. Therefore, as used herein the term "exhibiting symptoms of hyperactive sebaceous gland disorders" includes skin having symptoms and skin that is thought to be susceptible to developing symptoms in the future.

It is preferred that the affected subject receiving treatment is at least 12 years of age.

The present invention further relates to a topical hydrophobic or lipophilic photosensitizer composition for use in the treatment of a hyperactive sebaceous gland disorder selected from seborrhea, seborrheic dermatitis, and sebaceous gland hyperplasia wherein the photosensitizer is selected from verteporfin, lemuteporfin, and combinations thereof. In addition, the present invention relates to the use of a topical hydrophobic or lipophilic photosensitizer composition for the manufacture of a medicament for the treatment of a hyperactive sebaceous gland disorder selected from seborrhea, seborrheic dermatitis, and sebaceous gland hyperplasia wherein the photosensitizer is selected from verteporfin, lemuteporfin, and combinations thereof.

It has surprisingly been found that the hydrophobic or lipophilic photosensitizer compositions of the present invention can penetrate into the hair follicle and sebaceous gland but are only found at low levels in other, surrounding tissues. While not wishing to be bound by theory, it is believed that hydrophobic or lipophilic photosensitizer compositions better localize to the sebaceous glands thereby avoiding some of the severe side-effects reported in prior art treatments such as erythema, edema, pain, burning, and itching. It is believed that the selectivity of the present compositions avoids severe skin reactions and other adverse events. Furthermore the hydrophobic or lipophilic properties are thought to enable the photosensitizer to achieve high concentrations in the target organ, the sebaceous glands, that should translate in a greater efficacy, shorter photosensitizer application time, and/or the ability to use lower activation energy doses.

Preferred photodynamic treatment methods, compositions, and parameters are described in more detail below.

In one aspect the medicament is administered by a method involving:
(i) topically applying a hydrophobic and/or lipophilic photosensitizer composition to skin tissue exhibiting symptoms of a hyperactive sebaceous gland disorder selected from seborrhea, seborrheic dermatitis, and sebaceous gland hyperplasia,
(ii) removing excess composition from the skin, and
(iii) exposing the tissue to energy of a wavelength capable of activating the photosensitizer.

It has surprisingly been found that removing the excess photosensitizer does not compromise the efficacy of the treatment and may help avoid unwanted side effects.

The excess composition can be removed by any suitable method. Preferred methods include wiping with dry cloth, wiping with a moist towelette, washing with alcohol, washing with a soap free cleanser, washing with a mild soap cleanser, and combinations thereof. A preferred method of removing the excess composition is to wash the skin with mild shampoo such as Cliniderm.

Preferably the composition is left in contact with the skin for at least 1 minute, more preferably at least 5 minutes, even more preferably at least 15 minutes, before removal of excess.

Preferably the composition is left in contact with the skin for less than 120 minutes, more preferably less than 60 minutes, even more preferably less than 45 minutes, before removal of excess.

While not wishing to be bound by theory, it is also believed that the removal of the excess composition could avoid the photosensitizer in the excess creating a 'shadow' which would prevent the activation energy from reaching the target tissue (e.g. the sebaceous gland).

The treatment can be repeated any suitable number of times. It is preferred that at least two days, preferably at least five days, more preferably at least seven days, even more preferably at least ten days, even more preferably still fourteen days, are left between treatments.

In another aspect the activation energy is at least in part supplied by light emitting diodes. The LED's are preferably arrayed in a manner that somewhat follows the contours of the skin to be treated. A preferred arrangement is multiple flat panels of LED's that are moveable so that they can be positioned appropriately. One embodiment of this aspect of the invention involves the activation energy being delivered by an LED device that supplies both red (e.g. 600-750nm) and blue light (e.g. 390-450nm). A preferred embodiment supplies light at about 420nm and at about 690nm.

### Photodynamic Therapy

Preferably, the photosensitizer herein is delivered topically to the target tissue. Topical delivery avoids some of the photosensitivity issues associated with systemic delivery of photosensitizers. When the photosensitizer is applied topically it may be applied to the affected tissue alone or to the affected tissue and to unaffected tissues such as those surrounding the affected tissue.

The photosensitizer for use in the invention is selected from verteporfin, lemuteporfin, and combinations thereof.

Typically, these agents will absorb radiation in the range of from 400nm to 900nm, preferably from 450nm to 750nm, more preferably 500nm to 700nm.

As used herein, "photosensitizer" or "photosensitizing agent" means a chemical compound that absorbs electromagnetic radiation, most commonly in the visible spectrum, and releases it as energy, most commonly as reactive oxygen species and/or as thermal energy. Preferably, the compound is nontoxic to humans or is capable of being formulated in a nontoxic composition. Preferably, the chemical compound in its photodegraded form is also nontoxic. A nonexhaustive list of photosensitive chemicals maybe found in Kreimer-Birnbaum, Sem. Hematol. 26: 157-73, 1989 and in Redmond and Gamlin, Photochem. Photbiol. 70 (4): 391-475 (1999).

Preferred photosensitizers are those having a LogP value of 0 or greater. Preferably, the LogP of the photosensitizers herein is not less than 0.5, more preferably not less than 0.75, even more preferably not less than 1.0.

It is preferred that the photosensitizer composition used in the present methods is lipophilic.

Preferred photosensitizers are those having a molecular weight of 200g/mole or greater, more preferably 350g/mole or greater, even more preferably 500g/mole or greater. While not wishing to be bound by theory, it is believed that the higher molecular weight photosensitizers do not easily accumulate in non-target tissues such as the epidermis. This is believed to reduce the unwanted side effects such as pain.

Photosensitizers that strongly absorb light with extinction coefficients >10,000 M⁻¹cm⁻¹ are preferred.

There are a variety of synthetic and naturally occurring photosensitizers, including pro-drugs such as the pro-porphyrin 5-aminolevulinic acid (ALA) and derivatives thereof, porphyrins and porphyrin derivatives e.g. chlorins, bacteriochlorins, isobacteriochlorins, phthalocyanine and naphthalocyanines and other tetra- and polymacrocyclic compounds, and related compounds (e.g. pyropheophorbides, sapphyrins and texaphyrins) and metal complexes (such as tin, aluminum, zinc, lutetium). Other photosensitizers are tetrahydrochlorins, purpurins, porphycenes, and phenothiaziniums. Other photosensitizers include bacteriochlorophyll derivatives such as those described in WO-A-97/19081, WO-A-99/45382 and WO-A-01/40232. An exemplary bacteriochlorophyll is palladium bacteriopheophorbide WST09 (Tookad™). Furthermore photosensitizers include pro-porphyrim, porphyrins, and mixtures thereof. Some examples of pro-drugs include aminolevulinic acid such as Levulan™ and aminolevulinic acid esters such as described in WO-02/10120 and available as Metvix™, Hexvix™ and Benzvix™. Some examples of di-hydro or tetra-hydro porphyrins are described in EP-A 337,601 or WO-A-01/66550 and available as Foscan™ (temoporfin). The photosensitizer of the present invention is selected from verteporfin, lemuteporfin, and combinations thereof.

Under certain conditions it is preferred that the photosensitizers are selected from those which photobleach upon exposure to activation energy.

A particularly potent group of photosensitizers is known as green porphyrins, which are described in detail in U.S. Patent No. 5,171,749. The term "green porphyrins" refers to porphyrin derivatives obtained by reacting a porphyrin nucleus with an alkyne in a Diels-Alder type reaction to obtain a monohydrobenzoporphyrin. Such resultant macropyrrolic compounds are called benzoporphyrin derivatives (BPDs), which is a synthetic chlorin-like porphyrin with various structural analogues, as shown in U.S. Patent 5,171,749. Typically, green porphyrins are selected from a group of tetrapyrrolic porphyrin derivatives obtained by Diels-Alder reactions of acetylene derivatives with protoporphyrin under conditions that promote reaction at only one of the two available conjugated, nonaromatic diene structures present in the protoporphyrin-IX ring systems (rings A and B). Metallated forms of a Gp, in which a metal cation replaces one or two hydrogens in the center of the ring system, may also be used in the practice of the invention. The preparation of the green porphyrin compounds useful in this invention is described in detail in U.S. Patent No. 5,095,030,

Green porphyrins include benzoporphyrin derivative diester diacid (BPD-DA), mono-acid ring A (BPD-MA), mono-acid ring B (BPD-MB), or mixtures thereof. These compounds absorb light at about 692nm wavelength which has good tissue penetration properties. The compounds of formulas BPD-MA and BPD-MB may be homogeneous, in which only the C ring carbalkoxyethyl or only the D ring carbalkoxyethyl would be hydrolyzed or may be mixtures of the C and D ring substituent hydrolyzates.

Green porphyrins may also have the following general formula: 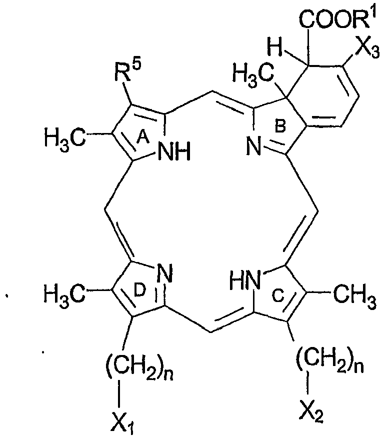 wherein; R⁵ is vinyl, R¹ and R⁶ are methyl, and n is 2. X₁, X₂, and X₃ are listed in the tables below:

**Table 1. Hydrophilic BPD B-zing analogs**

| **Drug** | **X₁** | **X₂** | **X₃** |
|---|---|---|---|
| QLT0061 | COOH | COOH | COOH |
| QLT0077 | CONH(CH₂)₂N+(CH₃)₃I. | CONH(CH₂)₂N+(CH₃)₃I- | COOCH₃ |
| QLT0079 | CONH(CH₂)₂N+(CH₃)₂((CH₂)₃CH₃ | CONH(H₂)₂N+(CH₃)₂((CH₂)₃CH₃) | COOCH₃ |
| QLT0086 | CONHCH(COOH)CH₂COOH | CONHCH(COOH)CH₂COOH | COOCH₃ |
| QLT0092 | CDNH(CH₂)₂NH(CH₃)₂ CF₃COO | CONH((CH₂)₂NH(CH₃)₂ CF₃COO- | COOCH₃ |
| QLT0094 | CDNHCH₂COOH | CONHCH₂COOH | CONHCH₂COOH |

**Table 2. Lipophilic BPD B-ring analogs**

| **Drug** | **X1** | **X2** | **X3** |
|---|---|---|---|
| QLT0060 | CO(O(CH)₂)0H | CO(O(CH₂)₂)0H | COOCH₃ |
| QLT0069 | COOCH₃ | COOCH₃ | COOH |
| QLT0078 | CO(C(CH₂)₂)₂0H | CO(O(CH₂)₂)₂0H | COOCH₃ |
| QLT0080 | CO(O(CH₂)₂)₃OH | COP(CH₂)₂)₃OH | COOCH₃ |
| QLT0081 | CO(O(CH₂)₂)₂OCH₃ | CO(O(CH₂)₂)₂OCH₃ | CO(O(CH₂)₂)₂OCH₃ |
| QLT0082 | CO(O(CH₂)₂)₂OH | CO(O(CH₂)₂)₂OH | CO(O(CH₂)₂)₂OH |
| QLT0083 | CO(O(CH₂)₂)₃OH | CO(O(CH₂)₂)₃OH | CO(O(CH₂)₂)₃OH |
| QLT0087 | CO(O(CH₂)₂)₄OH | CO(O(CH₂)₂)₄OH | COOCH₃ |
| QLT0088 | COOCH₃ | COOCH₃ | CONH(C₆H₄)(C₅H₁₀N |
| QLT0090 | CO(O(CH₂)₂)₅OH | CO(O(CH₂)₂)₅OH | COOCH₃ |
| QLT0093 | CO(O(CH₂)₂)₅OH | CO(O(CH₂)₂)₅OH | CO(O(CH₂)₂)₅OH |

The photosensitizers of the present invention are selected from verteporfin the benzoporphyrin derivative mono-acid (BPD-MA), lemuteporfin (QLT0074 as set forth in U.S. Pat. No. 5,929,105 referred to therein as A-EA6) and combinations thereof. A highly preferred photosensitizer is lemuteporfin which has the structure:

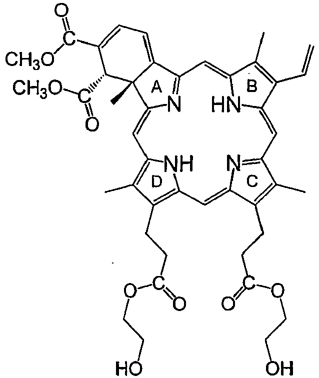

In addition to the above mentioned photosensitizing agents, other reference examples of photosensitizers include green porphyrins disclosed in US Pat. Nos. 5,283,255, 4,920,143, 4,883,790, 5,095,030, and 5,171,749; and green porphyrin derivatives, discussed in US Pat. Nos. 5,880,145 and 5,990,149. Several structures of typical green porphyrins are shown in the above cited patents, which also provide details for the production of the compounds.

The photosensitizers of the present invention, however, are selected from verteporfin, lemuteporfin, and combinations thereof.

The photosensitizer may be administered in any form suitable for topical application. The photosensitizer may be used alone or as components of mixtures. For example, the photosensitizer may be administered by means including lotions, ointments, creams, pastes, or suspensions. Preferred are lotions, creams, and ointments.

The photosensitizers may be formulated into a variety of compositions. These compositions may comprise any component that is suitable for the intended purpose, such as conventional delivery vehicles and excipients including isotonising agents, pH regulators, solvents, solubilizers, dyes, gelling agents and thickeners and buffers and combinations thereof. Pharmaceutical formulations suitable for use with the instant photosensitizers can be found, for instance, in Remington's Pharmaceutical Sciences. Preferred formulations herein comprise pharmaceutical excipients or carriers capable of directing the photosensitizer to the sebaceous gland. Suitable excipients for use with photosensitizers include water, saline, dextrose, and glycerol .

Typically, the photosensitizer is formulated by mixing it, at an appropriate temperature, e.g., at ambient temperatures, and at appropriate pHs, and the desired degree of purity, with one or more physiologically acceptable carriers, i.e., carriers that are nontoxic at the dosages and concentrations employed.

Preferred formulations are described in WO03/39597. The formulations preferably comprise a skin-penetration enhancer. Any skin-penetration enhancer suitable for aiding the delivery of the photosensitizing agent can be used herein. A list of skin-penetration enhancers can be found in "Pharmaceutical Skin Penetration Enhancement" (1993) Walters, K.A., ed.; Hadgraft, J., ed - New York, N.Y. Marcel Dekker and in "Skin Penetration Enhancers cited in the Technical Literature" Osbourne, D.W. Pharmaceutical Technology, November 1997, pp 59-65.

Preferred for use in the formulations herein are hydrophobic skin-penetration enhancers. Preferred skin-penetration enhancers are selected from glycol ethers, fatty acids, fatty acid esters, glycol esters, glycerides, azones, polysorbates, alcohols, dimethylsulfoxide, and mixtures thereof. Preferred skin-penetration enhancers for use herein include diethylene glycol monoethyl ether (Transcutol®), Oleyl alcohol, Oleic acid, Azone (Laurocapram or 1-n-Dodecyl azacycloheptan-2-one), Propylene glycol mono- and diesters of fats and fatty acids (e.g. propylene glycol monocaprylate, propylene glycol monolaurate), Triglycerides and lipids (e.g. linoleic acid), Macrogolglycerides or Polyethylene glycol glycerides and fatty esters (e.g. stearoyl macrogolglycerides, oleoyl macrogolglycerides, lauroyl macrogolglycerides, Oleyl macrogol-6-glycerides, Lauroyl macrogol-6 glycerides), Glycerides and fatty acid esters of polyethylene glycol (e.g. caprylocaproyl macrogolglycerides, capryl-caproyl macrogolglycerides, oleoyl macrogol glycerides), Polyoxyl 40 Hydrogenated Castor Oil (Cremophor RH 40), Polysorbate 80 (Tween 80), Dodecylazacycloheptanone, SEPA® such as described in US Patent 4,861,764 (e.g. 2-n-nonyl-1,3-dioxolane), and mixtures thereof. More preferred is diethylene glycol monoethyl ether (available from Gattefosse under the tradename Transcutol).

It is preferred that the formulations comprise from about 0.1% to about 99%, preferably from about 0.1% to about 90%, more preferably from about 5% to about 90%, even more preferably from about 15% to about 75%, by weight of skin penetration enhancer.

It is preferred that the ratio of photosensitizer to skin-penetration enhancer is from about 1:20 to about 1:10000, more preferably from about 1:60 to 1:300, on the basis of percentages by weight of total composition.

It is preferred that the photosensitizer is solubilised, especially when the photosensitizer is hydrophobic or lipophilic. One method of solubilising certain photosensitizers, including green porphyrins, is by formulation in liposomes or other lipid-containing complexes. An alternative may be to solubilise the photosensitizer in cyclodextrins or cyclodextrin derivatives. Preferred are partially etherified cyclodextrin, the ether substituents of which are hydroxyethyl, hydroxypropyl or dihydroxypropyl groups. However, appropriate cyclodextrins should be of a size and conformation appropriate for use with the photosensitizing agents disclosed herein.

A hydrophilic photosensitizer can also be formulated into a hydrophobic carrier formulation by, for example, encapsulating the photosensitizer in liposomes.

Other methods suitable for solubilising certain photosensitizers include the use of a solvent acceptable for use in the treatment of skin tissues and cells such as, but are not limited to, DMSO (dimethylsulfoxide), polyethylene glycol (PEG) or any other solvent. It is preferred that the formulations herein comprise a solubilizer. Some solubilizers are also penetration enhancers and it is preferred that the formulations herein comprise a penetration enhancer that is also a solubilizer for the photosensitizer. Preferably the solubilizer is selected from glycol ethers, polyethylene glycol, polyethylene glycol derivatives, propylene glycol, propylene glycol derivatives, polysorbates (e.g. Tween™), fatty alcohols, aromatic alcohols, propylene glycol, glycerols, oils, surfactants, glucosides, and mixtures thereof. More preferably the solubilizer is selected from diethylene glycol monoethyl ether (Transcutol), polyethylene glycol of average molecular weight from 100 to 5000, triethylene glycol, tetraethylene glycol, pentaethylene glycol, hexaethylene glycol, septaethylene glycol, octaethylene glycol, propylene glycol, propylene glycol mono- and diesters of fats and fatty acids (e.g. propylene glycol monocaprylate, propylene glycol monolaurate), benzyl alcohol, glycerol, oleyl alcohol, mineral oil, lanolin/lanolin derivatives, petrolatum or other petroleum products suitable for application to the skin, propylene glycol mono- and diesters of fats and fatty acids, macrogols, macrogolglycerides or polyethylene glycol glycerides and fatty esters (e.g. stearoyl macrogolglycerides, oleoyl macrogolglycerides, lauroyl macrogolglycerides, linoleoyl macrogolglycerides), ethoxylated castor oil (e.g. Cremophor - a polyoxyl hydrogenated castor oil), C6-C30 triglycerides, natural oils, glucosides (e.g. cetearyl glucoside), surfactants, and mixtures thereof. More preferable the solubilizer is selected from diethylene glycol monoethyl ether (Transcutol), oleyl alcohol, and mixtures thereof.

It is preferred that the formulations herein comprise from about 0.1% to about 99%, more preferably from about 1% to about 75%, by weight of solubilizer.

It is preferred that the formulations have a viscosity at 20°C of from about 50 cps to about 50000 cps, more preferably from about 500 cps to about 40000 cps, even more preferably from about 5000 cps to about 30000 cps. Should the viscosity need to be adjusted it can be done by means of a viscosity modifying agent. Preferred viscosity modifiers are selected from polyethylene glycols, acrylic acid-based polymers (carbopol polymers or carbomers), polymers of acrylic acid crosslinked with allyl sucrose or allylpentaerythritol (carbopol homopolymers), polymers of acrylic acid modified by long chain (C10-C30) alkyl acrylates and crosslinked with allylpentaerythritol (carbopol copolymers), poloxamers also known as pluronics (block polymers; e.g. Poloxamer 124, 188, 237, 338, 407), waxes (paraffin, glyceryl monostearate, diethylene glycol monostearate, propylene glycol monostearate, ethylene glycol monosterate, glycol stearate), hard fats (e.g. Saturated C8-C18 fatty acid glycerides), xantham gum, polyvinyl alcohol, solid alcohols, and mixtures thereof.

Preferred formulations contain one or more PEGs. It is preferred that the formulation comprises at least one PEG of average molecular weight about 2000 or less, preferably about 1500 or less, preferably about 1000 or less, preferably about 800 or less, preferably about 600 or less, preferably about 500 or less, preferably about 400 or less. It is preferred that the formulation comprises at least one PEG of average molecular weight about 3000 or more, preferably about 3350 or more, preferably about 3500 or more. It is preferred that the formulation comprises a mixture of PEG's. More preferably, one PEG has an average molecular weight of about 800 or less and one PEG has an average molecular weight of 3000 or more.

A preferred formulation for use in the present invention comprises photosensitizer verteporfin, lemuteporfin and/or combinations thereof), low molecular weight PEG such as PEG200, diethylene glycol monoethyl ether (Transcutol), high molecular weight PEG such as PEG3350 and fatty alcohol such as oleyl alcohol.

The formulation herein may comprise a variety of other components. Any suitable ingredient may be used herein but typically these optional components will render the formulations more cosmetically acceptable or provide additional usage benefits. Some examples of preferred optional ingredients include emulsifiers, humectants, emollients, surfactants, oils, waxes, fatty alcohols, dispersants, skin-benefit agents, pH adjusters, dyes/colourants, analgesics, perfumes, preservatives, and mixtures thereof.

Preparation of dry formulations that are reconstituted immediately before use also is contemplated. The preparation of dry or lyophilized formulations can be effected in a known manner, conveniently from the solutions of the invention. The dry formulations of this invention are also storable. By conventional techniques, a solution can be evaporated to dryness under mild conditions, especially after the addition of solvents for azeotropic removal of water, typically a mixture of toluene and ethanol. The residue is thereafter conveniently dried, e.g. for some hours in a drying oven.

For topical formulations (such as ointments) to be applied to the surface of the skin, the concentration of the photosensitizer in the excipient preferably ranges from about 0.001 to about 10% w/w, and more preferably from about 0.005 to about 5% w/w, and even more preferably between about 0.01 to about 1% w/w. Particularly preferred is the use of about a 02% w/w topical formulation.

Preferably sufficient time is left between delivery of the photosensitizer and administration of the activation energy to allow the photosensitizer to distribute within the target tissue. The exact length of time can vary according to the type of photosensitizer and the target tissue but, in general, it is preferred that 10 seconds or more, more preferably 1 minute or more, more preferably 5 minutes or more, is left between delivery of the photosensitizer and administration of the activation energy Preferably the time between delivery of the drug and activation energy is 240 minutes or less, more preferably 180 minutes or less, even more preferably 60 minutes or less. While not wishing to be bound by theory, shorter contact times are thought to be preferably because there is less time for the photosensitizer to accumulate in non-target tissues and a consequent reduction in the incidence and/or severity of side-effects.

Preferably the photosensitizer is delivered in a topical composition and is left in contact with the skin for 5 to 60 minutes. If necessary, excess composition can be preferably removed by any suitable means. Preferred means include wiping with dry cloth, wiping with a moist towelette, washing with alcohol, washing with a soap free cleanser, washing with a mild soap cleanser, and combinations thereof. Thereafter, it is preferred that the activation energy is delivered to the skin. This period will vary depending on the photosensitizer and the method of delivery. For example, lemuteporfin delivered topically can be activated shortly after application whereas ALA requires a delaywhile the ALA is metabolized into the photosensitive active.

Preferably, the activation energy comprises a wavelength close to at least one of the absorption peaks of the photosensitizer. This wavelength differs for different photosensitizers. For example, BPD-MA has an absorption peak at 689nm and so, when BPD-MA is the photosensitizer used, the wavelength of the activation energy is preferably is at or close to 689nm. The photosensitizer ALA-methyl ester (available under the tradename Metvix) has an absorption peak at 635nm and so when this photosensitizer is used the activation energy is preferable at or close to 635nm ALA (available under the tradename Levulan) has an absorption peak at 417nm and at 630nm so when this photosensitizer is used the activation energy is preferable at or close to 417nm and/or 630nm. The activation energy herein may be provided by any suitable means. Generally, the activation energy is provided by a visible light source although it has been suggested that x-ray, ultraviolet, or ultrasound sources may be used. Preferred sources include lasers, light emitting diodes (LED), incandescent lamps, arc lamps, standard fluorescent lamps, U.V. lamps, and combinations thereof. More preferred are light emitting diodes. Alternatively, any convenient source of activation energy having a component of wavelengths that are absorbed by the photosensitizer may be used, for example, an operating room lamp, or any bright light source, including sunlight. Commercially available activation energy sources include CureLight™ (available from Photocure ASA, Oslo, Norway), BLU-U™ (available from DUSA, Wilmington, MA, USA), PDT Laser (available from Diomed, Andover, MA, USA), Ceralas™ (available from Biolitec AG, Jena, Germany), Omnilux PDT™ (available from PhotoTherapeutics Ltd., Birmingham, UK), and Q-Beam & Quanta-med (Quantum Devices Inc., Barneveld, WI, USA).

The activation energy dose administered during the PDT treatment contemplated herein can vary as necessary. Preferably, for photosensitizers of high potency, such as green porphyrins, the dosage of the light is about 25-100 J/cm². It is generally preferred that the total dose of the irradiation should generally not exceed 400 J/cm², preferably 200 J/cm², or more preferably not exceed 100 J/cm². Preferred doses can range between about 0.1 J/cm² to about 200 J/cm², more preferably 1 J/cm² to about 100 J/cm². For example, about 25, about 50, about 75, about 100, about 125, about 150, or about 175 J/cm². More preferred doses range from about 25 J/cm² to about 100 J/cm².

Normally, the intensity of the energy source should not exceed about 600mW/cm². Irradiances between about 0.1 and 400 mW/cm² are preferred. Even more preferably the irradiance is between 5 and 100 mW/cm².

Normally, the irradiation lasts from about 10 seconds to about 4 hours, and preferably between about 5 minutes and 1 hour. For example, irradiation times of about 10, about 15, about 20, about 30, about 45, about 60, about 75, about 90, about 105, about 120, about 135, about 150, about 165 and about 180 minutes may be used.

It is preferred that the area to be treated have minimal hair coverage when the activation energy is applied. Therefore, if there is significant hair coverage of the area to be treated, it is preferred that the hair is cut short or shaved prior to activation energy application. While not wishing to be bound by theory, it is believed that, due to the fact that hair has a shielding function, hair coverage can affect the activation energy dose that is delivered to the target area, especially when visible light wavelengths are used. Consequently, in order to more accurately deliver the correct does it is preferred that there be little or no hair coverage. Alternatively, the shielding effect of the hair may be compensated for by changes to delivery of the activation energy.

The irradiation or light exposure used in the invention may be directed to a small or large area of the body or face depending on the patch to be treated. Any part of the body may be treated but acne typically affects the face, chest, and/or back Treatment may be preceded with an assessment of the time of light exposure for the patient's minimal erythemal dose (MED) occurrence in order to avoid potential burning of the exposed skin.

The treatment maybe repeated as many times as is necessary. If repeated, the treatment frequency may vary. For example, the treatments could be daily, every two days, twice weekly, weekly, every two weeks, twice monthly, every four weeks, monthly, every six weeks, every eight weeks, every two months, quarterly, twice annually, or annually, or other suitable time interval. Preferably the treatment is not repeated more than once per week, even more preferably not more than once every two weeks. Preferably, the treatment is repeated at least once every six months. More preferably at least once every three months. Even more preferably at least once every two months. The total number of treatments can range from one to as many as required. It is preferred that the total number of treatments in any 6 month period be from 1 to 12, more preferably from 1 to 6, even more preferably from 2 to 3.

A preferred regimen according to the present invention comprises:
a) administering lipophilic photosensitizer composition topically to the affected skin. Preferably the composition comprises photosensitizer and skin-penetration enhancer. The photosensitizer is selected from verteporfin, lemuteporfin, or combinations thereof.
b) removing excess composition, preferably with a moist towelette.
c) administering activation energy, preferably via LED's. Preferably, the activation energy is administered within 60 minutes of application. Preferred doses are between 15 and 200 J/cm². More preferred doses include 20, 40, 80, or 120 J/cm²_{.}

### EXAMPLES

It will be understood that the following embodiments of the present invention are intended to be illustrative of some of the possible applications or principles.

### Example 1

The effect of different red light doses on the mouse sebaceous glands in response to PDT was evaluated. Lemuteporfin ointment was applied to shaved flank skin of male Balb/c mice and left on for 30 min. Excess material was removed from the skin surface and the site was exposed to a 688-nm red light dose of 25, 50, 100, 200 or 400 J/cm² delivered at an intensity of 50 mW/cm². Three days later, mice were euthanized and full-thickness skin excised from the PDT treatment site as well as the untreated contra-lateral side. Skin samples were processed, sectioned and stained for lipid using Oil Red O. Numbers of Oil Red O-positive pilosebaceous units (PSU) per 4x microscopic image were determined by two independent readers. At all light doses the number of Oil Red O-positive PSU was less for the PDT-treated sites than for untreated sites.

### Example 2

Patients with moderate to severe acne as defined by the presence of pustular and or cystic lesions, with or without scarring, are assessed for PDT treatment with lemuteporfin 0.2% ointment. Prior to treatment, the areas to be treated with PDT are cleansed and cleared of any hair, skin lotions or cosmetic products.

Topical photosensitizer ointment (comprising 0.2 wt% lemuteporfin, 50 wt% PEG-200, 24 wt% Transcutol^{®} , 10wt% PEG-3350 and 15.8 wt% oleyl alcohol) is applied directly on the acne affected skin at a quantity of approximately 45 mg / cm2. The ointment is left on for absorption for 20-45 minutes. Immediately prior to light treatment, excess ointment is removed by gentle wiping with a water-based skin cleanser. Acne lesions and the immediate surrounding areas are illuminated with 689 nm PDT light at a dose of 100J/cm2 with an intensity of 50mW/cm2.

## Claims

1. Use of a hydrophobic and/or lipophilic photosensitizer composition in the manufacture of a medicament for the treatment of a hyperactive sebaceous gland disorder selected from seborrhea, seborrheic dermatitis, and sebaceous gland hyperplasia, wherein said medicament is administered by a method comprising:
(i) topically applying said medicament to skin tissue exhibiting symptoms of a hyperactive sebaceous gland disorder selected from seborrhea, seborrheic dermatitis, and sebaceous gland hyperplasia, and
(ii) exposing the tissue to energy at a wavelength capable of activating the photosensitizer, wherein the photosensitizer is selected from verteporfin, lemuteporfin, and combinations thereof.

2. A use according to Claim 1, wherein the photosensitizer is lemuteporfin.

3. A use according to Claim 1 or 2, wherein the composition has a viscosity at 20°C of from about 50 cps to about 50000 cps.

4. A use according to any one of the preceding Claims, wherein excess photosensitizer composition is removed from the skin prior to application of activation energy.

5. A use according to any one of the preceding claims, wherein the treatment is repeated at least once every three months or six months.

6. A use according to any one of the preceding claims, wherein at least 5 days is left before the treatment is repeated.

7. A use according to any one of the preceding claims, wherein the activation energy is at least in part supplied by a light emitting diode device.

8. A use according to Claim 7, wherein the activation energy is at least in part supplied by a light emitting diode device wherein the device emits red and blue light.

9. A hydrophobic and/or lipophilic photosensitizer composition for use in the treatment of a hyperactive sebaceous gland disorder selected from seborrhea, seborrheic dermatitis, and sebaceous gland hyperplasia, wherein the photosensitizer is selected from verteporfin, lemuteporfin, and combinations thereof.

10. A hydrophobic and/or lipophilic photosensitizer composition for use according to Claim 9, wherein the photosensitizer is lemuteporfin.

11. Use of a hydrophobic and/or lipophilic photosensitizer composition in the preparation of a medicament for the treatment of a hyperactive sebaceous gland disorder selected from seborrhea, seborrheic dermatitis, and sebaceous gland hyperplasia, wherein the photosensitizer is selected from verteporfin, lemuteporfin, and combinations thereof.

12. A use according to Claim 11, wherein the photosensitizer is lemuteporfin.

## Patentansprüche

1. Verwendung einer hydrophoben und/oder lipophilen photosensibilisierenden Zusammensetzung bei der Herstellung eines Medikaments zur Behandlung einer Hyperaktivitätsstörung der Talgdrüsen, die ausgewählt ist aus Seborrhoe, seborrhoischer Dermatitis und einer Hyperplasie der Talgdrüsen, wobei das Medikament mittels eines Verfahrens verabreicht wird, welches umfasst:
(i) das topische Aufbringen des Medikaments auf Hautgewebe, auf dem sich Symptome einer Hyperaktivitätsstörung der Talgdrüsen, die ausgewählt ist aus Seborrhoe, seborrhoischer Dermatitis und einer Hyperplasie der Talgdrüsen, manifestieren, und
(ii) das Exponieren des Gewebes gegenüber Energie mit einer Wellenlänge, die zu einer Aktivierung des Photosensibilisators in der Lage ist, wobei der Photosensibilisator ausgewählt ist aus Verteporfin, Lemuteporfin und Kombinationen aus diesen.

2. Verwendung nach Anspruch 1, wobei der Photosensibilisator Lemuteporfin ist.

3. Verwendung nach Anspruch 1 oder 2, wobei die Zusammensetzung bei 20°C eine Viskosität von zwischen etwa 50 cps und etwa 50.000 cps aufweist.

4. Verwendung nach einem der vorhergehenden Ansprüche, wobei ein Überschuss der photosensibilisierenden Zusammensetzung vor der Anwendung von Aktivierungsenergie von der Haut entfernt wird.

5. Verwendung nach einem der vorhergehenden Ansprüche, wobei die Behandlung mindestens einmal alle drei Monate oder sechs Monate wiederholt wird.

6. Verwendung nach einem der vorhergehenden Ansprüche, wobei vor einer Wiederholung der Behandlung mindestens 5 Tage gewartet wird.

7. Verwendung nach einem der vorhergehenden Ansprüche, wobei die Aktivierungsenergie zumindest zum Teil durch eine LED (*light emitting diode*)-Vorrichtung zur Verfügung gestellt wird.

8. Verwendung nach einem der vorhergehenden Ansprüche, wobei die Aktivierungsenergie zumindest zum Teil durch eine LED *(light emitting diode*)-Vorrichtung zur Verfügung gestellt wird, wobei die Vorrichtung rotes und blaues Licht emittiert.

9. Hydrophobe und/oder lipophile photosensibilisierende Zusammensetzung zur Verwendung bei der Behandlung einer Hyperaktivitätsstörung der Talgdrüsen, die ausgewählt ist aus Seborrhoe, seborrhoischer Dermatitis und einer Hyperplasie der Talgdrüsen, wobei der Photosensibilisator ausgewählt ist aus Verteporfin, Lemuteporfin und Kombinationen aus diesen.

10. Hydrophobe und/oder lipophile photosensibilisierende Zusammensetzung zur Verwendung nach Anspruch 9, wobei der Photosensibilisator Lemuteporfin ist.

11. Verwendung einer hydrophoben und/oder lipophilen photosensibilisierenden Zusammensetzung bei der Herstellung eines Medikaments zur Behandlung einer Hyperaktivitätsstörung der Talgdrüsen, die ausgewählt ist aus Seborrhoe, seborrhoischer Dermatitis und einer Hyperplasie der Talgdrüsen, wobei der Photosensibilisator ausgewählt ist aus Verteporfin, Lemuteporfin und Kombinationen aus diesen.

12. Verwendung nach Anspruch 11, wobei der Photosensibilisator Lemuteporfin ist.

## Revendications

1. Utilisation d'une composition photosensibilisante hydrophobe et/ou lipophile dans la fabrication d'un médicament destiné au traitement d'un trouble des glandes sébacées hyperactives choisi parmi la séborrhée, la dermite séborrhéique, et l'hyperplasie des glandes sébacées, où ledit médicament est administré par un procédé comprenant :
(i) l'application topique dudit médicament sur le tissu cutané présentant des symptômes d'un trouble des glandes sébacées hyperactives choisi parmi la séborrhée, la dermite séborrhéique, et l'hyperplasie des glandes sébacées, et
(ii) l'exposition du tissu à une énergie à une longueur d'onde capable d'activer le photosensibilisant, où le photosensibilisant est choisi parmi la vertéporfine, la lémutéporfine, et leurs combinaisons.

2. Utilisation selon la revendication 1, où le photosensibilisant est la lémutéporfine.

3. Utilisation selon la revendication 1 ou 2, où la composition présente une viscosité à 20°C d'environ 50 cps à environ 50000 cps.

4. Utilisation selon l'une quelconque des revendications précédentes, où l'excès de composition photosensibilisante est éliminé de la peau avant l'application d'une énergie d'activation.

5. Utilisation selon l'une quelconque des revendications précédentes, où le traitement est répété au moins une fois tous les trois mois ou six mois.

6. Utilisation selon l'une quelconque des revendications précédentes, où il est laissé au moins 5 jours avant la répétition du traitement.

7. Utilisation selon l'une quelconque des revendications précédentes, où l'énergie d'activation est au moins en partie fournie par un dispositif de diode électroluminescente.

8. Utilisation selon la revendication 7, où l'énergie d'activation est au moins en partie fournie par un dispositif de diode électroluminescente où le dispositif émet de la lumière rouge et bleue.

9. Composition photosensibilisante hydrophobe et/ou lipophile pour une utilisation dans le traitement d'un trouble des glandes sébacées hyperactives choisi parmi la séborrhée, la dermite séborrhéique, et l'hyperplasie des glandes sébacées, où le photosensibilisant est choisi parmi la vertéporfine, la lémutéporfine, et leurs combinaisons.

10. Composition photosensibilisante hydrophobe et/ou lipophile pour une utilisation selon la revendication 9, où le photosensibilisant est la lémutéporfine.

11. Utilisation d'une composition photosensibilisante hydrophobe et/ou lipophile dans la préparation d'un médicament destiné au traitement d'un trouble des glandes sébacées hyperactives choisi parmi la séborrhée, la dermite séborrhéique, et l'hyperplasie des glandes sébacées, où le photosensibilisant est choisi parmi la vertéporfine, la lémutéporfine, et leurs combinaisons.

12. Utilisation selon la revendication 11, où le photosensibilisant est la lémutéporfine.
